# EUROPEAN PATENT APPLICATION

(11) **EP 2 372 392 A1**
(43) Date of publication of application: **05.10.2011**
(21) Application number: 11160233.0
(22) Date of filing: 29.03.2011
(51) Int. Cl.: G01S 15/89, G06T 3/20, G06T 7/20, A61B 8/08

(54) **Ultrasound diagnosis apparatus, image processing apparatus, and image processing method**

(30) Priority: 31.03.2010 JP 2010081030; 31.01.2011 JP 2011017988
(71) Applicant: Kabushiki Kaisha Toshiba, Tokyo 105-8001 (JP); Toshiba Medical Systems Corporation, Otawara-shi, Tochigi-ken 324-8550 (JP)
(72) Inventor: Kamiyana, Naohisa, Otawara-Shi, Tochigi 324-8550 (JP)
(74) Representative: Kramer - Barske - Schmidtchen

(57) **Abstract**

According to one embodiment, an ultrasound diagnosis apparatus includes an image creating unit (15a), a motion-vector calculating unit (15b), a corrected-image creating unit (15c), and a control unit (17). The image creating unit (15a) creates a plurality of ultrasound images in time series; and the motion-vector calculating unit (15b) calculates a motion vector of a local region between two successive ultrasound images (a first image and a second image) in time series among the ultrasound images created by the image creating unit (15a). The corrected-image creating unit (15c) then creates a corrected image corrected from the second image, based on a component of a scanning line direction of ultrasound in the motion vector calculated by the motion-vector calculating unit (15b). The control unit (17) then performs control so as to cause a certain monitor (2) to display the corrected image created by the corrected-image creating unit (15c).

## Description

### FIELD

Embodiments described herein relate generally to an ultrasound diagnosis apparatus, an image processing apparatus, and an image processing method.

### BACKGROUND

Conventionally, an ultrasound diagnosis apparatus is small in size of equipment, compared with other medical diagnostic imaging apparatuses, such as an X-ray diagnosis apparatus, an X-ray Computed Tomography (CT) apparatus, and a Magnetic Resonance Imaging (MRI) apparatus; and moreover, it can display in real time a state of movement of an examination subject, for example, a heartbeat or a motion of a fetus, with an easy operation only by touching an ultrasound probe to the body surface; therefore, it plays an important role in today's medical practice. Furthermore, among ultrasound diagnosis apparatuses with no fear of radiation exposure, an apparatus that is downsized small enough to carry by one hand has been developed; so that such ultrasound diagnosis apparatus can be easily used in medical practices, such as a department of obstetrics and home care.

Moreover, development of a technology of objectively and quantitatively analyzing a state of tissue of an examination subject by using an ultrasound image created by an ultrasound diagnosis apparatus has been recently in progress. As such technology, a tissue tracking imaging method, an ultrasound elastography method, and the like are known.

The tissue tracking imaging method is a technology of, for example, tracking the position of a myocardium following beat and then integrating signals derived from velocity information about the myocardium, thereby creating and displaying a short-axis image of the heart that depicts displacement and distortion of the myocardium, for performing functional analysis of a heart (for example, see JP-A 2005-124636 (KOKAI)).

The ultrasound elastography method is a technology of creating and displaying an image that can provide an objective and quantitative analysis for a malignancy diagnosis of a tumoral lesion (specifically, hardness), which has been performed by observing a B-mode image by a doctor.

A malignancy diagnosis of a tumoral lesion by B-mode image observation is explained below. The malignancy diagnosis of a tumoral lesion by B-mode image observation is a typical diagnosis using real-time display by an ultrasound diagnosis apparatus, and specifically, a manipulation explained below is performed. When a tumoral lesion is discovered on a B-mode image, a doctor or an engineer slightly presses and releases an affected area with an ultrasound probe in touch with a body surface. When performing such manipulation, living body tissue including the tumor is deformed. At that time, when it is observed such that the tumoral lesion is displaced in parallel along with the press and release by the ultrasound probe, the doctor can conclude that the tumoral lesion is hard.

On the other hand, when change in the shape of the tumoral lesion is observed along with a press by the ultrasound probe, for example, it is flattened from a spherical shape, the doctor can conclude that the tumoral lesion is soft. Because sometimes the shape of a tumoral lesion can be originally flat in some cases, when change is observed such that the shape of a tumoral lesion is further flattened along with a press by the ultrasound probe, the doctor can also conclude that the tumoral lesion is soft.

In this way, for performing a malignancy diagnosis of a tumoral lesion, it is useful to observe change in shape of a tumoral lesion by using a real-time display (moving image display) function of the ultrasound diagnosis apparatus. Such diagnosis is routinely performed, for example, during an ultrasound image diagnosis of breast cancer.

On the other hand, the ultrasound elastography method is a technology of calculating-deformation of living body tissue including a tumoral lesion in accordance with small change in the phase of ultrasound pulse, thereby reconstructing and displaying a two-dimensional mapping image of tissue distortion. As a method of deforming living body tissue including a tumoral lesion, in addition to the method of pressing and releasing with an ultrasound probe as described above, a method of pressing with a mechanical effect of an ultrasound pulse (for example, Acoustic Radiation Force Impulse (ARFI)) is known.

As described above, the ultrasound elastography method is a technology of measuring the modulus of elasticity of living body tissue including a tumoral lesion and imaging it; however, sometimes there is a gap between a diagnosis result by using a two-dimensional mapping image displayed by the ultrasound elastography method, and an empirical diagnosis result obtained by actually observing B-mode image, in some cases. A liquid, such as water, is a substance with very small compressibility; however, a liquid wrapped by a film easily deforms. For this reason, when a tumor includes a liquid in its inside, a measuring error of the modulus of elasticity becomes large. Moreover, also when there is a blood vessel or a blood flow in the vicinity of living body tissue including a tumoral lesion, a measuring error of the modulus of elasticity becomes large. As described above, there is a gap between "softness", i.e., "deformative tendency" that has been conventionally experienced in clinical practice, and a modulus of elasticity as a physical constant.

For such reasons, it is difficult to use a two-dimensional mapping image reconstructed by the ultrasound elastography method as an ultimate evidence for a malignancy diagnosis of a tumoral lesion, and under the present circumstances, a malignancy diagnosis of a tumoral lesion is often performed through observation of B-mode image while performing press and release with an ultrasound probe.

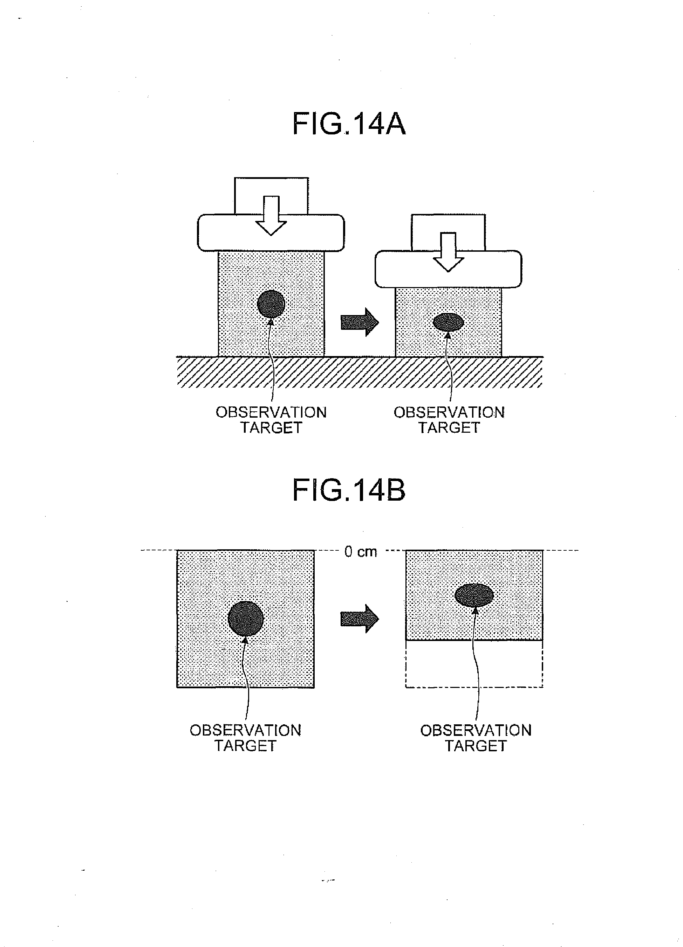

Apart from that, there is a problem that visibility of an observation target on a B-mode image (ultrasound image) is degraded through the above-described observation of a B-mode image, due to a fundamental display method of an ultrasound diagnosis apparatus. FIGS. 14A and 14B are schematic diagrams for explaining a problem of a conventional technology.

Usually, while a stationary object is in a state of being pressed from its top, when looking at the state of the object from the lateral side, as shown in FIG. 14A, the object is compressed downward, and a portion of an observation target inside the object is also moved downward. In such case, the ultrasound diagnosis apparatus displays an ultrasound image that the surface of an ultrasound probe is constantly at zero centimeter. Accordingly, observing the ultrasound image, as shown in FIG. 14B, the living body tissue is compressed upward, and the observation target (tumor) inside the living body tissue is moved upward. In other words, the living body tissue and the observation target (tumor) are drawn on the ultrasound image as they look like moving in the direction opposite to the actual movement direction, consequently, it is difficult for an observer of the ultrasound image to grasp a state of movement of the observation target. For this reason, to grasp a state of movement of an observation target that moves in the opposite direction to an actual movement direction, the observer is required to improve observation techniques.

Moreover, the observation target is deformed and displaced in parallel due to press and release by the ultrasound probe. Consequently, the observer needs to observe the degree of deformation while deformation and displacement is simultaneously occurring, sometimes resulting in having an illusion in some cases. The above description explains that there is a problem that visibility of an observation target is degraded, when observing an ultrasound image while performing press and release with an ultrasound probe during a malignancy diagnosis of a tumoral lesion. However, even when generally observing living body tissue under a body surface by touching an ultrasound probe to the body surface of a subject, the body surface of the subject is pressed along with movement of the ultrasound probe, and living body tissue of an observation target sometimes moves in some cases. In other words, according to the fundamental display method of the ultrasound diagnosis apparatus described above, not only during a malignancy diagnosis of a tumoral lesion, but also during diagnostic imaging using ultrasound images, there is a problem that visibility of an observation target on an ultrasound image is degraded.

### BRIEF DESCRIPTION OF THE DRAWINGS

FIG. 1 is a schematic diagram for explaining an ultrasound diagnosis apparatus according to a first embodiment;
FIG. 2 is a schematic diagram for explaining a configuration of an image processing unit according to the first embodiment;
FIG. 3 is a schematic diagram for explaining a monitoring Region Of Interest (ROI);
FIGS. 4 and 5 are schematic diagrams for explaining a corrected-image creating unit;
FIG. 6 is a flowchart for explaining processing by the ultrasound diagnosis apparatus according to the first embodiment;
FIG. 7 is a schematic diagram for explaining a configuration of an image processing unit according to a second embodiment;
FIG. 8 is a schematic diagram for explaining an observation ROI;
FIG. 9 is a schematic diagram for explaining a motion-vector calculating unit and a deformation-rate calculating unit according to the second embodiment;
FIGS. 10A, 10B, and 10C are schematic diagrams for explaining the deformation-rate calculating unit and a control unit according to the second embodiment;
FIG. 11 is a flowchart for explaining processing by the ultrasound diagnosis apparatus according to the second embodiment;
FIG. 12 is a schematic diagram for explaining a first modification of the second embodiment;
FIG. 13 is a schematic diagram for explaining a second modification of the second embodiment; and
FIGS. 14A and 14B are schematic diagrams for explaining a problem of a conventional technology.

### DETAILED DESCRIPTION

According to one embodiment, an ultrasound diagnosis apparatus includes an image creating unit, a calculating unit, a corrected-image creating unit, and a display control unit. The image creating unit creates a plurality of ultrasound images in time series based on a reflected wave of ultrasound that is transmitted onto a subject from an ultrasound probe. The calculating unit calculates a motion vector of a local region between a first image and a second image that are two successive ultrasound images in time series among the ultrasound images created by the image creating unit. The corrected-image creating unit creates a corrected image corrected from the second image, based on a component of a scanning line direction of the ultrasound in the motion vector calculated by the calculating unit. The display control unit performs control so as to cause a certain display unit to display the corrected image created by the corrected-image creating unit.

Exemplary embodiments of an ultrasound diagnosis apparatus will be explained below in detail with reference to the accompanying drawings.

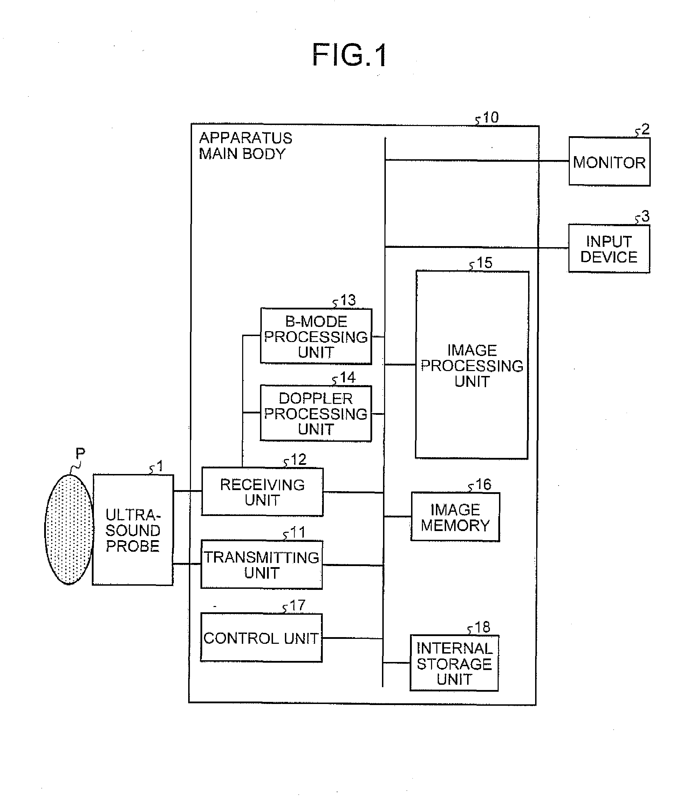

First of all, a configuration of an ultrasound diagnosis apparatus according to a first embodiment is explained below. FIG. 1 is a schematic diagram for explaining the ultrasound diagnosis apparatus according to the first embodiment. As shown in FIG. 1, the ultrasound diagnosis apparatus according to the first embodiment includes an ultrasound probe 1, a monitor 2, an input device 3, and an apparatus main body 10.

The ultrasound probe 1 includes a plurality of piezoelectric vibrators; and the piezoelectric vibrators generate ultrasound based on a driving signal supplied from a transmitting unit 11 included in the apparatus main body 10, which will be described later, and furthermore, receives a reflected wave from the subject P and converts it into an electric signal. Moreover, the ultrasound probe 1 includes a matching layer provided to the piezoelectric vibrators, and a backing material that prevents propagation of ultrasound backward from the piezoelectric vibrators.

When ultrasound is transmitted from the ultrasound probe 1 to the subject P, the transmitted ultrasound is consecutively reflected by discontinuity planes of acoustic impedance in internal body tissue of the subject P, and received as a reflected wave signal by the piezoelectric vibrators included in the ultrasound probe 1. The amplitude of the received reflected wave signal depends on a difference in the acoustic impedance of the discontinuity planes that reflect the ultrasound. A reflected wave signal when a transmitted ultrasound pulse is reflected by a moving blood flow or a surface of a heart wall is affected by a frequency deviation, dependently on a velocity component in the ultrasound transmitting direction of a moving object, due to the Doppler effect.

The monitor 2 displays a Graphical User Interface (GUI) for an operator of the ultrasound diagnosis apparatus to input various setting requests by using the input device 3, and displays an ultrasound image created by the apparatus main body 10.

The input device 3 includes a mouse, a keyboard, a button, a panel switch, a touch command screen, a foot switch, a trackball, and the like; receives various setting requests from an operator of the ultrasound diagnosis apparatus; and transfers each of the received various setting requests to the apparatus main body 10 (for example, a request to set a region of interest). The input device 3 according to the first embodiment includes a processing execution switch for receiving from the operator a start and an ending of image processing to be performed by an image processing unit 15, which will be described later.

The apparatus main body 10 is a device that creates an ultrasound image based on a reflected wave received by the ultrasound probe 1; and includes the transmitting unit 11, a receiving unit 12, a B-mode processing unit 13, a doppler processing unit 14, the image processing unit 15, an image memory 16, a control unit 17, and an internal storage unit 18, as shown in FIG. 1.

The transmitting unit 11 includes a trigger generating circuit, a delay circuit, a pulsar circuit, and the like; and supplies a driving signal to the ultrasound probe 1. The pulsar circuit repeatedly generates a rate pulse for forming transmission ultrasound at a certain rate frequency. The delay circuit gives to each rate pulse generated by the pulsar circuit, a delay time with respect to each of the piezoelectric vibrators to be used for converging ultrasound generated from the ultrasound probe 1 into a beam and determining transmission directivity. The trigger generating circuit applies a driving signal (driving pulse) to the ultrasound probe 1 at timing based on the rate pulse.

The receiving unit 12 includes an amplifier circuit, an analog/digital (A/D) converter, an adder, and the like; and creates reflected wave data by performing various kinds of processing on a reflected wave signal received by the ultrasound probe 1. The amplifier circuit performs gain correction processing by amplifying the reflected wave signal. The A/D converter converts from analog to digital the reflected wave signal of which gain is corrected, and gives a delay time required for determining reception directivity. The adder creates reflected wave data by performing addition processing of the reflected wave signal processed by the A/D converter. Through the addition processing by the adder, a reflection component from a direction in accordance with the reception directivity of the reflected wave signal is emphasized.

In this way, the transmitting unit 11 and the receiving unit 12 control transmission directivity and reception directivity in transmission and reception of ultrasound.

The B-mode processing unit 13 receives reflected wave data from the receiving unit 12; performs logarithmic amplification, envelope detection processing, and the like; and creates data (B-mode data) that a signal strength is expressed by the brightness.

The doppler processing unit 14 performs frequency analysis on velocity information from the reflected wave data received from the receiving unit 12; extracts components of a blood flow, tissue, and contrast media echo by Doppler effects; and creates data (doppler data) that moving object information, such as an average velocity, a distribution, a power, and the like, are extracted with respect to multiple points.

The image processing unit 15 creates an ultrasound image from B-mode data created by the B-mode processing unit 13 or doppler data created by the doppler processing unit 14. Specifically, the image processing unit 15 creates a B-mode image from B-mode data, and a doppler image from doppler data. Moreover, the image processing unit 15 converts (scan-converts) a scanning-line signal sequence of an ultrasound scan into a scanning-line signal sequence in a video format typified by television, and creates an ultrasound image (a B-mode image or a doppler image) as a display image. Furthermore, the image processing unit 15 performs image processing that will be described later in detail on the created ultrasound image.

The image memory 16 is a memory that stores an ultrasound image created by the image processing unit 15, and an image created by performing image processing on an ultrasound image by the image processing unit 15.

The control unit 17 controls processing by the ultrasound diagnosis apparatus overall. Specifically, the control unit 17 controls processing performed by the transmitting unit 11, the receiving unit 12, the B-mode processing unit 13, the doppler processing unit 14, and the image processing unit 15, based on various setting requests input by the operator via the input device 3 and various control programs and various setting information read from the internal storage unit 18. Moreover, the control unit 17 performs control so as to display on the monitor 2 an ultrasound image stored by the image memory 16, and the like.

The internal storage unit 18 stores various data, such as: control programs for performing ultrasound transmission and reception, image processing, and display processing; diagnosis information (for example, a patient ID, a doctor's opinion); a diagnosis protocol; and various setting information. Moreover, the internal storage unit 18 is used for storing images stored in the image memory 16, as required. Data stored by the internal storage unit 18 can be transferred to an external peripheral device via a not shown interface circuit.

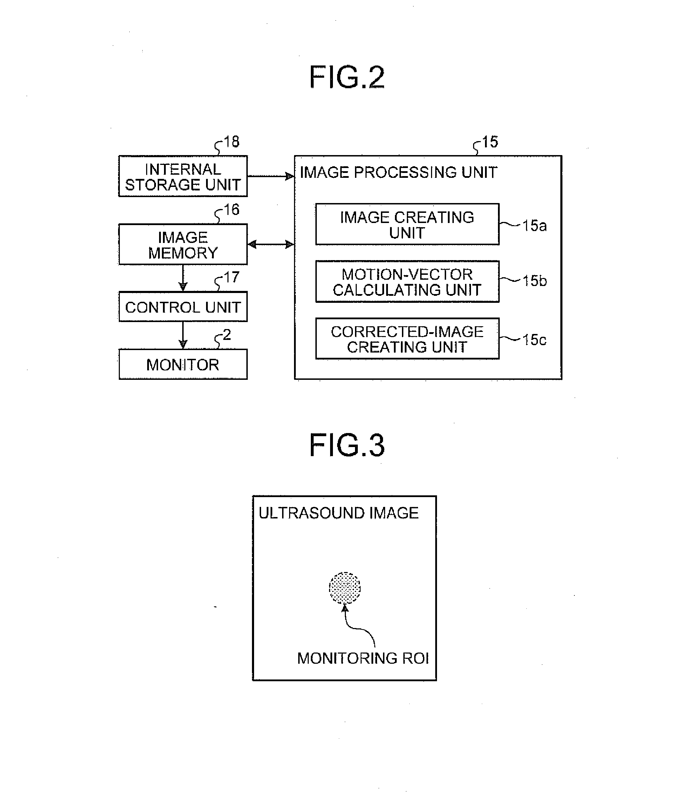

In this way, the ultrasound diagnosis apparatus according to the first embodiment creates an ultrasound image based on a reflected wave of ultrasound transmitted from the ultrasound probe 1; and it is configured to be capable to improve visibility of an observation target on the ultrasound image through processing performed by the image processing unit 15, which will be explained below in detail. Image processing to be executed by the image processing unit 15 according to the first embodiment is explained below in detail with reference to FIG. 2. The following description explains a case where during an examination of breast cancer, a tumoral lesion is discovered on a B-mode image that a breast of the subject P is image, consequently, the degree of malignancy of the tumoral lesion is diagnosed by referring to ultrasound images displayed along a time sequence while a doctor or an engineer is pressing and releasing the breast of the subject P with the ultrasound probe 1 in touch with the breast.

FIG. 2 is a schematic diagram for explaining a configuration of the image processing unit according to the first embodiment. As shown in FIG. 2, the image processing unit 15 according to the first embodiment includes an image creating unit 15a, a motion-vector calculating unit 15b and a corrected-image creating unit 15c.

The image creating unit 15a creates a B-mode image from B-mode data, and a doppler image from doppler data, as an ultrasound image. The image creating unit 15a then stores the created ultrasound image into the image memory 16. Specifically, according to the first embodiment, the image creating unit 15a creates a plurality of B-mode images in time series from a plurality of B-mode data sequentially created by the B-mode processing unit 13 along a time sequence when a doctor or an engineer sequentially presses and releases a breast of the subject P with the ultrasound probe 1 in touch with the breast.

Each time when the image creating unit 15a sequentially creates a B-mode image along the time sequence and newly stores it into the image memory 16, the control unit 17 then sequentially reads the newly stored B-mode image from the image memory 16, and causes the monitor 2 to display it.

At that time, if the operator turns ON the "processing execution switch" included in the input device 3, the control unit 17 controls the image processing unit 15 so as to start image processing by the motion-vector calculating unit 15b and the corrected-image creating unit 15c.

To begin with, the motion-vector calculating unit 15b calculates a motion vector of a local region between two successive ultrasound images in time series, among a plurality of ultrasound images created by the image creating unit 15a. Specifically, the motion-vector calculating unit 15b calculates a motion vector between a local region of a new image and a local region of an ultrasound image created immediately before the new image, each time when a new image that is a new ultrasound image is created by the image creating unit 15a.

A local region in the embodiment is a Region Of Interest (ROI) that is preliminarily set for monitoring a motion vector, and hereinafter described as a "monitoring ROI". According to the embodiment, setting information about the position of a monitoring ROI, the size of the monitoring ROI, and the shape of the monitoring ROI are preliminarily stored by the internal storage unit 18. FIG. 3 is a schematic diagram for explaining a monitoring ROI.

For example, in a case of characterization of a tumoral lesion of a breast cancer, as shown in FIG. 3, the motion-vector calculating unit 15b sets the position of a monitoring ROI at the center of an ultrasound image (B-mode image) in accordance with setting information preliminarily stored by the internal storage unit 18, and sets the size and the shape of the monitoring ROI for example, to a perfect circle five millimeters in diameter. Although the first embodiment is explained below in a case where setting information about a monitoring ROI is preliminarily stored by the internal storage unit 18, it can be alternatively in a case where such information is set each time of diagnosis by an operator who refers to the ultrasound image.

The following description explains a case where processing by the motion-vector calculating unit 15b is to be executed between an image "i" and an image "i+1", which are two successive ultrasound images in time series. The motion-vector calculating unit 15b calculates a motion vector between an image "i" and an image "i+1" by identifying similarity between an image pattern in a monitoring ROI on the image "i" and an image pattern in a monitoring ROI on the image "i+1".

An existing algorithm can be applied as a calculating method for a motion vector. However, because of characteristics of moving-image display in real time by the ultrasound diagnosis apparatus, the motion-vector calculating unit 15b needs to perform processing of, for example, approximately 30 frames per second. For this reason, the motion-vector calculating unit 15b uses, for example, a method of minimizing the sum of Absolute Differences (SAD) of brightness values, as an algorithm that enables fast processing.

In other words, the motion-vector calculating unit 15b calculates the SAD by slightly moving the position of the monitoring ROI of the image "i+1" upward/downward and leftward/rightward. Specifically, the motion-vector calculating unit 15b calculates an absolute value of a brightness difference between the brightness value (pixel value) of each pixel in the moved monitoring ROI and the brightness value of each pixel in the monitoring ROI of the image "i" between corresponding pixels, and then calculates the total sum of the calculated absolute values (SAD). The motion-vector calculating unit 15b then calculates a motion vector "vector V(i+1)" of the image "i+1" with respect to the image "i", from the position of the monitoring ROI of the image "i+1" and the position of the monitoring ROI of the image "i" when the calculated SAD becomes the minimum.

Through such processing, the motion-vector calculating unit 15b sequentially calculates a motion vector "vector V(2)" of an image "2" with respect to an image "1", a motion vector "vector V(3)" of an image "3" with respect to the image "2", a motion vector "vector V(4)" of an image "4" with respect to the image "3", and the like.

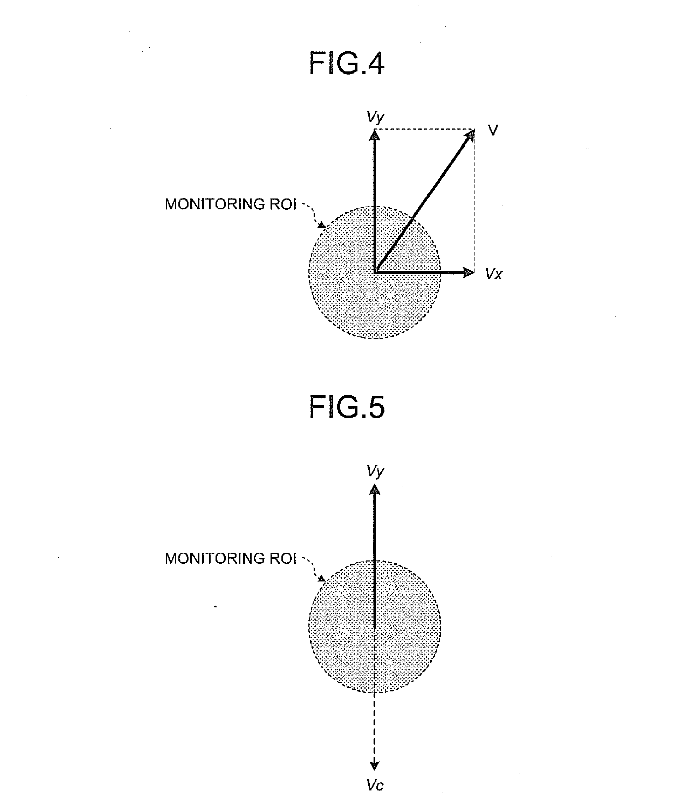

Based on a component of a scanning line direction of ultrasound in the motion vector calculated by the motion-vector calculating unit 15b, the corrected-image creating unit 15c then creates a corrected image that is corrected from an ultrasound image created at the latter time among two ultrasound images (an image "i+1" that is a new image created subsequently to an image "i"). FIGS. 4 and 5 are schematic diagrams for explaining the corrected-image creating unit.

To begin with, as shown in FIG. 4, the corrected-image creating unit 15c separates the motion vector "vector V" calculated by the motion-vector calculating unit 15b into a "vector Vy" that is a component of a scanning line direction of ultrasound (the vertical component with respect to the vibrator plane of the ultrasound probe 1), and a "vector Vx" that is a component orthogonal to the component of the scanning line direction of ultrasound (the parallel component with respect to the vibrator plane of the ultrasound probe 1).

The corrected-image creating unit 15c then calculates a "vector Vc" that is the same in magnitude as the "vector Vy", and opposite in direction to the "vector Vy", as shown in FIG. 5. The corrected-image creating unit 15c then creates a corrected image corrected from the new image, based on the calculated "vector Vc", and stores the created corrected image into the image memory 16.

For example, the corrected-image creating unit 15c calculates a "vector V(2)y" that is the vertical component of the motion vector "vector V(2)" of the image "2" with respect to the image "1", and then calculates an inverse vector "vector V(2)c" of the "vector V(2)y". The corrected-image creating unit 15c then creates a corrected image "2" by moving the image "2" by the "vector V(2)c".

Moreover, when processing the image "3", the corrected-image creating unit 15c calculates a "vector V(3)y" that is a vertical component of the motion vector "vector V(3)" of the image "3" with respect to the image "2", and calculates an inverse "vector V(3)c" of the "vector V(3)y". The corrected-image creating unit 15c then creates a corrected image "3" by moving the image "3" by '"vector V(2)c"+"vector V(3)c"'.

Furthermore, when processing the image "4", the corrected-image creating unit 15c calculates a "vector V(4)y" that is a vertical component of the motion vector "vector V(4)" of the image "4" with respect to the image "3", and calculates an inverse "vector V(4)c" of the "vector V(4)y". The corrected-image creating unit 15c then creates a corrected image "4" by moving the image "4" by '"vector V(2)c"+"vector V(3)c"+"vector V(4)c"'. On such corrected images, the position of the monitoring ROI in the vertical direction is substantially constant.

When creating a corrected image, the corrected-image creating unit 15c according to the first embodiment determines whether to create a corrected image through threshold processing that is explained below. In other words, if a magnitude of the vertical component of a motion vector is equal to or larger than a threshold (for example, two millimeters) preliminarily stored by the internal storage unit 18, the corrected-image creating unit 15c creates a corrected image from an ultrasound image subjected to corrected image creation (an image "i+1" that is a new image). By contrast, if a magnitude of the vertical component of a motion vector is smaller than the threshold, the corrected-image creating unit 15c determines not to create corrected image from the image "i+1".

The motion-vector calculating unit 15b can calculate a motion vector "vector V(i+1)" of the monitoring ROI between a corrected image "i" and an image "i+1". In such case, the corrected-image creating unit 15c creates a corrected image "i+1" corrected from the image "i+1" by moving the image "i+1" by a "vector Vc(i+1)" that is an inverse vector of the vertical component of "vector V(i+1)".

The control unit 17 shown in FIGS. 1 and 2 reads a corrected image created by the corrected-image creating unit 15c from the image memory 16, and performs control so as to cause the monitor 2 to display the read corrected image. When corrected image is not created from an image "i+1" by the corrected-image creating unit 15c, the control unit 17 reads the image "i+1" from the image memory 16, and causes the monitor 2 to display it.

According to such display control processing, the monitor 2 animatedly displays ultrasound images (B-mode images) on which the position of the monitoring ROI in the vertical direction is substantially constant.

The control unit 17 performs control such that as the operator turns ON the "processing execution switch", the processing by the motion-vector calculating unit 15b and the corrected-image creating unit 15c is to be executed, as described above. The control unit 17 then performs control such that as the operator turns OFF the "processing execution switch", the processing by the motion-vector calculating unit 15b and the corrected-image creating unit 15c is to be stopped.

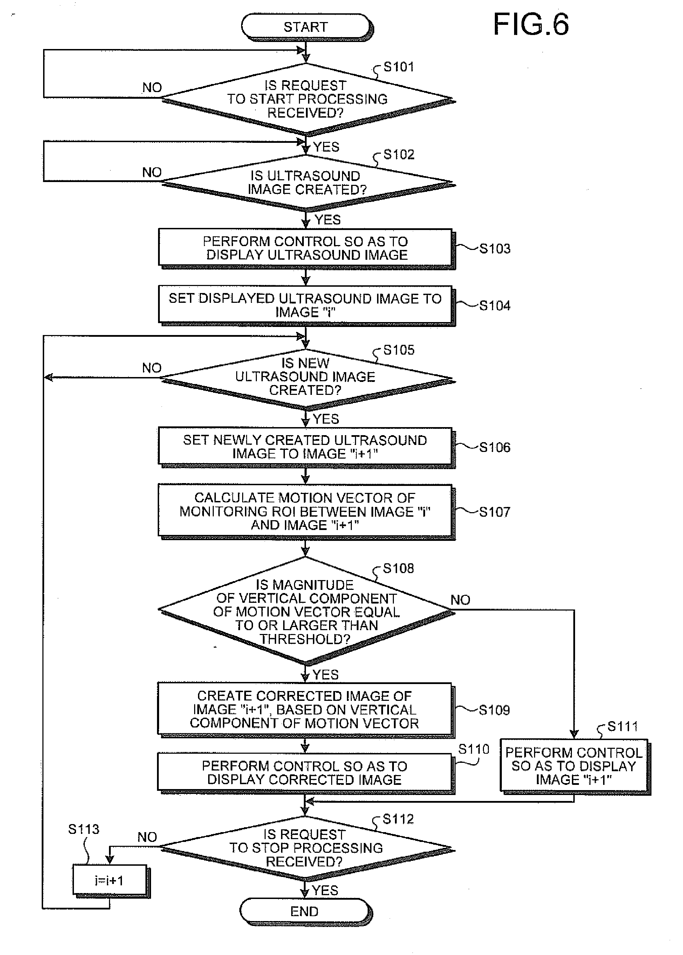

Processing by the ultrasound diagnosis apparatus according to the first embodiment is explained below with reference to FIG. 6. FIG. 6 is a flowchart for explaining the processing by the ultrasound diagnosis apparatus according to the first embodiment. With FIG. 6, processing to be performed after preliminarily setting a monitoring ROI and a threshold those are used for creation of a corrected image is explained below.

As shown in FIG. 6, the ultrasound diagnosis apparatus according to the first embodiment determines whether the operator turns ON the "processing execution switch" included in the input device 3 and a request to start the processing is received (Step S101). If the request to start the processing is not received (No at Step S101), the ultrasound diagnosis apparatus turns into a standby state.

By contrast, if the request to start the processing is received (Yes at Step S101), the control unit 17 determines whether an ultrasound image is created by the image creating unit 15a (Step S102). If ultrasound image is not created (No at Step S102), the control unit 17 is on standby until an ultrasound image is created.

By contrast, if an ultrasound image is created (Yes at Step S102), the control unit 17 performs control so as to display the created ultrasound image on the monitor 2 (Step 5103); and sets the displayed image to an image "i" (Step S104).

The control unit 17 then determines whether a new ultrasound image is created (Step S105); if new image is not created (No at Step S105), the control unit 17 is on standby until a new ultrasound image is created.

By contrast, if a new ultrasound image is created (Yes at Step S105), the control unit 17 sets the newly created ultrasound image to an image "i+1" (Step S106); and the motion-vector calculating unit 15b calculates a motion vector of the monitoring ROI between the image "i" and the image "i+1" (Step S107).

The corrected-image creating unit 15c then determines whether a magnitude of the vertical component of the motion vector calculated by the motion-vector calculating unit 15b is equal to or larger than the threshold (Step S108). If the magnitude of the vertical component of the motion vector is smaller than the threshold (No at Step S108), the control unit 17 performs control so as to display the image "i+1" on the monitor 2 (Step S111).

By contrast, if the magnitude of the vertical component of the motion vector is equal to or larger than the threshold (Yes at Step S108), the corrected-image creating unit 15c creates a corrected image of the image "i+1" based on the vertical component of the motion vector (Step S109). The control unit 17 then performs control so as to display the corrected image corrected from the image "i+1" on the monitor 2 (Step S110).

After that, the control unit 17 determines whether the operator turns OFF the "processing execution switch" included in the input device 3, and a request to stop the processing is received (Step S112). If the request to stop the processing is not received (No at Step S112), the control unit 17 sets the image "i+1" to an image "i" (Step S113), returns to Step S105, and determines whether a new ultrasound image is created. In other words, the control unit 17 performs control such that the processing is to be executed between the ultrasound image set as an image "i" at Step S113, and an image "i+1" that is a new image set as an image "i+1" at Step S106.

By contrast, if the request to stop the processing is received (Yes at Step S112), the control unit 17 terminates the processing by the motion-vector calculating unit 15b and the corrected-image creating unit 15c.

As described above, according to the first embodiment, the image creating unit 15a creates a plurality of ultrasound images in time series; and the motion-vector calculating unit 15b calculates a motion vector of a monitoring ROI between two successive ultrasound images in time series, among the ultrasound images created by the image creating unit 15a. Based on the component of the scanning line direction of ultrasound in the motion vector calculated by the motion-vector calculating unit 15b, the corrected-image creating unit 15c then creates a corrected image that is corrected from an ultrasound image created at the later time of two ultrasound images. The control unit 17 then performs control so as to cause the monitor 2 to display the corrected image created by the corrected-image creating unit 15c.

Consequently, according to the first embodiment, the position of an observation target on a displayed image can be substantially constant in the vertical direction, so that visibility of the observation target on the ultrasound image can be improved. Moreover, according to the first embodiment, because correction processing is performed based on the component of the scanning line direction of ultrasound in a motion vector, for example, even when the operator moves the ultrasound probe 1 along a body surface in order to observe another portion, it can avoid performing the correction processing with the horizontal component of the motion vector, which is not required for the operator.

Furthermore, according to the first embodiment, the corrected-image creating unit 15c creates a corrected image when a magnitude of the vertical component of a motion vector is equal to or larger than a threshold; and when the corrected-image creating unit 15c does not create corrected image, the control unit 17 causes the monitor 2 to display an image subjected to image processing. Consequently, according to the first embodiment, when only a small movement is detected, creation processing of unnecessary corrected image can be avoided, so that a load related to image processing can be reduced.

Moreover, according to the first embodiment, when receiving a request to execute processing from the operator, the motion-vector calculating unit 15b and the corrected-image creating unit 15c execute the calculation processing of motion vector and the creation processing of corrected image, respectively; and stop the calculation processing of motion vector and the creation processing of corrected image when receiving a request to stop the processing from the operator. Therefore, according to the first embodiment, execution of the correction processing on a displayed image only during a period desired by the operator can be achieved. According to the first embodiment, because a corrected image is created based on the vertical component of a motion vector; even if the operator moves the ultrasound probe 1 along a body surface in order to observe another portion while the "processing execution switch" is ON, it can avoid performing the correction processing with the horizontal component of the motion vector, which is not required for the operator.

The above processing can be executed in a case of generally observing living body tissue under a body surface by applying the ultrasound probe 1 onto the body surface of the subject P, in addition to the case of observing a tumoral lesion of a breast.

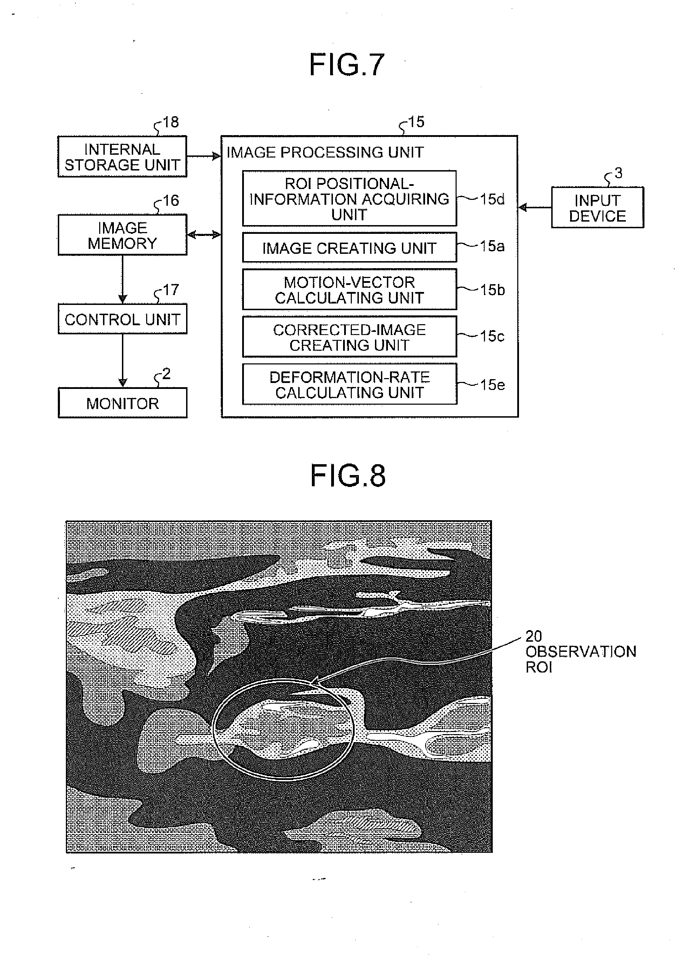

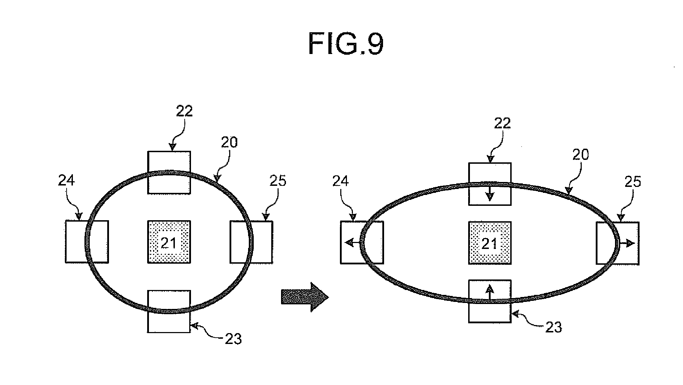

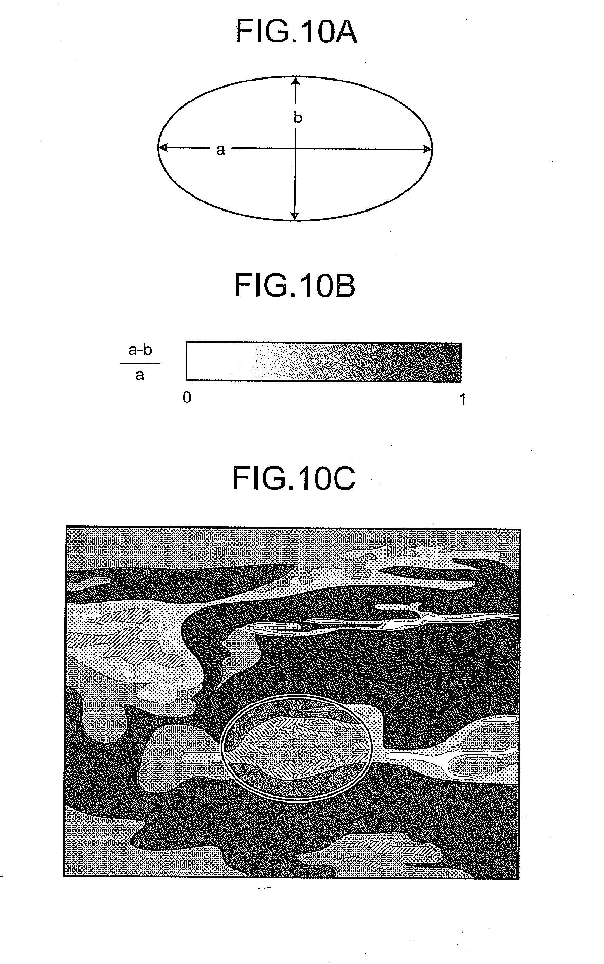

Although a case of using one monitoring ROI is explained in the first embodiment, a case of using a plurality of monitoring ROIs is explained below in a second embodiment with reference to FIGS. 7, 8, 9, 10A, 10B, and 10C. FIG. 7 is a schematic diagram for explaining a configuration of an image processing unit according to the second embodiment; FIG. 8 is a schematic diagram for explaining an observation ROI; FIG. 9 is a schematic diagram for explaining a motion-vector calculating unit and a deformation-rate calculating unit according to the second embodiment; and FIGS. 10A, 10B, and 10C are schematic diagrams for explaining the deformation-rate calculating unit and a control unit according to the second embodiment.

The ultrasound diagnosis apparatus according to the second embodiment is configured similarly to the ultrasound diagnosis apparatus according to the first embodiment explained with reference to FIG. 1. However, compared with the image processing unit 15 according to the first embodiment explained with reference to FIG. 2, the image processing unit 15 according to the second embodiment is different in the point that an ROI positional-information acquiring unit 15d and a deformation-rate calculating unit 15e are further included, as shown in FIG. 7. The following description explains mainly about these.

To begin with, according to the second embodiment, the operator sets a region of an observation target on a B-mode image as a region of interest for observation (observation ROI). For example, the operator sets an observation ROI 20 by roughly tracing the contour of a tumoral lesion observed on a B-mode image, as shown in FIG. 8, by using a drawing function that the input device 3 includes. According to the example shown in FIG. 8, the observation ROI 20 is drawn in ellipse on a B-mode image.

The ROI positional-information acquiring unit 15d acquires positional information about the observation ROI 20 drawn on the B-mode image. The motion-vector calculating unit 15b according to the second embodiment then sets a plurality of monitoring ROIs, based on positional information on the B-mode image of the observation ROI 20 acquired by the ROI positional-information acquiring unit 15d.

For example, the motion-vector calculating unit 15b sets a monitoring ROI 21 at a substantial center of the observation ROI 20, as shown in the left figure in FIG. 9. Here, the monitoring ROI 21 is to be used for calculating a motion vector for creation of a corrected image described above. The motion-vector calculating unit 15b then sets, for example, monitoring ROIs 22 to 25 at four positions on the border of the observation ROI 20, as shown in the left figure in FIG. 9. According to the example shown in the left figure in FIG. 9, the monitoring ROIs 22 to 25 are set at respective four positions that are two end points of the long side and two end points of the short side in the observation ROI 20 in ellipse. In this example, the monitoring ROIs 22 to 25 are to be used for calculating a motion vector for analyzing a state of deformation of the drawn observation ROI 20.

The motion-vector calculating unit 15b executes setting of a plurality of monitoring ROIs, for example, based on setting information prestored by the internal storage unit 18. Alternatively, setting of a plurality of monitoring ROIs can be executed by the operator who refers to the B-mode image, together with the setting of an observation ROI. Although the shape of each monitoring ROI is a square in the example shown in the left figure in FIG. 9, it can be a case where the shape of each monitoring ROI is a perfect circle, as explained in the first embodiment. In this way, according to the second embodiment, the monitoring ROIs 21 to 25 are set as a plurality of local regions, in the observation ROI 20 that is drawn on an ultrasound image. Hereinafter, the monitoring ROI 21 is sometimes described as a "first local region", and the monitoring ROIs 22 to 25 are sometimes described as local regions other than the first local region".

Under such state, the operator turns ON the "processing execution switch", and presses and releases the body surface of a breast of the subject P by using the ultrasound probe 1.

The motion-vector calculating unit 15b then calculates respective motion vectors of the monitoring ROIs 21 to 25 between two successive ultrasound images in time series (an image "i" and an image "i+1"), among a plurality of ultrasound images created by the image creating unit 15a. For example, the motion-vector calculating unit 15b calculates a motion vector by calculating an SAD, which is explained above in the first embodiment.

The corrected-image creating unit 15c then creates a corrected image by using a motion vector of the monitoring ROI 21 (first local region) calculated by the motion-vector calculating unit 15b. In other words, the corrected-image creating unit 15c creates an image "i+1", based on the vertical component of the motion vector of the monitoring ROI 21.

Also according to the second embodiment, when a magnitude of the vertical component of the motion vector of the monitoring ROI 21 is equal to or larger than the threshold, the corrected-image creating unit 15c creates a corrected image of the image "i+1"; and when a magnitude of the vertical component of the motion vector of the monitoring ROI 21 is smaller than the threshold, it is determined not to create corrected image of the image "i+1". Accordingly, the corrected-image creating unit 15c creates a corrected image on which the position of the monitoring ROI 21 in the vertical direction is substantially constant, similarly to the first embodiment.

The deformation-rate calculating unit 15e shown in FIG. 7 calculates a deformation rate of the observation ROI 20, based on the motion vectors of the monitoring ROIs 22 to 25, which are local regions other than the first local region. For example, the deformation-rate calculating unit 15e calculates a deformation rate of the observation ROI 20, based on the motion vector of the monitoring ROI 21, and the motion vectors of the monitoring ROIs other than the monitoring ROI 21 (the monitoring ROIs 22 to 25).

Specifically, the deformation-rate calculating unit 15e calculates a relative vector between the vertical component of the motion vector of the monitoring ROI 21 and the vertical component of the motion vector of the monitoring ROI 22, thereby calculating an amount of movement of the monitoring ROI 22 in the vertical direction. Moreover, the deformation-rate calculating unit 15e calculates a relative vector between the vertical component of the motion vector of the monitoring ROI 21 and the vertical component of the motion vector of the monitoring ROI 23, thereby calculating an amount of movement of the monitoring ROI 23 in the vertical direction.

Furthermore, the deformation-rate calculating unit 15e calculates a relative vector between the horizontal component of the motion vector of the monitoring ROI 21 and the horizontal component of the motion vector of the monitoring ROI 24, thereby calculating an amount of movement of the monitoring ROI 24 in the horizontal direction. Moreover, the deformation-rate calculating unit 15e calculates a relative vector between the horizontal component of the motion vector of the monitoring ROI 21 and the horizontal component of the motion vector of the monitoring ROI 25, thereby calculating an amount of movement of the monitoring ROI 25 in the horizontal direction.

Accordingly, the deformation-rate calculating unit 15e can calculate a deformation rate indicating that, for example, the observation ROI 20 is expanded in the horizontal direction, and reduced in size in the vertical direction, as shown in the right figure in FIG. 9.

Specifically, as shown in FIG. 10A, the deformation-rate calculating unit 15e calculates a length "a" of the long side of the observation ROI 20 on the image "i+1" as a deformation rate, from the amounts of movement of the monitoring ROI 24 and the monitoring ROI 25 in the horizontal direction. Similarly, as shown in FIG. 10A, the deformation-rate calculating unit 15e calculates a length "b" of the short side of the observation ROI 20 on the image "i+1" as a deformation rate, from the amounts of movement of the monitoring ROI 22 and the monitoring ROI 23 in the vertical direction.

Furthermore, the deformation-rate calculating unit 15e calculates a compression rate "(a-b)/a" of the observation ROI on the image "i+1". In other words, because the observation ROI is an ellipse, the compression rate is a useful value as an index indicating the deformation rate of the observation ROI.

The control unit 17 causes the monitor 2 to display a corrected image created from the image "i+1" or the image "i+1" similarly to the first embodiment; and when displaying it, the control unit 17 causes a display image to reflect information about the deformation rate of the observation ROI calculated by the deformation-rate calculating unit 15e.

In other words, the control unit 17 performs control so as to deform the observation ROI in an image to be displayed on the monitor 2, based on the deformation rate of the observation ROI ("a" and "b") calculated by the deformation-rate calculating unit 15e.

Furthermore, the control unit 17 performs control so as to change the color of the observation ROI in an image to be displayed on the monitor 2 to a certain color. Specifically, the control unit 17 performs control so as to change the color of the observation ROI, based on a color scale that color tones vary in accordance with a magnitude of the compression rate "(a-b)/a" calculated by the deformation-rate calculating unit 15e, as shown in FIG. 10B. The control unit 17 performs control so as to color the observation ROI to translucent so that a displayed image can be referred to.

Owing to the above control by the control unit 17, as shown in FIG. 10C, the monitor 2 displays a corrected image "i+1" or the image "i+1" superimposed with the observation ROI colored into a color tone in accordance with the compression rate and deformed by the deformation rate.

The display image that reflects the deformation rate described above can be created, for example, through the processing by the corrected-image creating unit 15c and the image creating unit 15a.

The control unit 17 then performs control such that as the operator turns OFF the "processing execution switch", the processing by the motion-vector calculating unit 15b, the corrected-image creating unit 15c, and the deformation-rate calculating unit 15e is to be stopped.

The deformation-rate calculating unit 15e can calculate a deformation rate of the observation ROI 20 based on only the motion vectors of the monitoring ROIs 22 to 25, without using the motion vector of the monitoring ROI 21. In such case, the deformation-rate calculating unit 15e calculates a relative vector between the vertical component of the motion vector of the monitoring ROI 22 and the vertical component of the motion vector of the monitoring ROI 23, thereby calculating an amount of movement of the observation ROI 20 in the vertical direction. Moreover, the deformation-rate calculating unit 15e calculates a relative vector between the horizontal component of the motion vector of the monitoring ROI 24 and the horizontal component of the motion vector of the monitoring ROI 25, thereby calculating an amount of movement of the observation ROI 20 in the horizontal direction. This also enables the deformation-rate calculating unit 15e to calculate the length "a" of the long side of the observation ROI 20 and the length "b" of the short side of the observation ROI 20, shown in FIG. 10A. Moreover, owing to this, according to the second embodiment, as shown in FIG. 10C, it can display an image on which the shape and the color of an observation ROI vary in accordance with the values (the length of the long side, the length of the short side, and the compression rate) calculated by the deformation-rate calculating unit 15e.

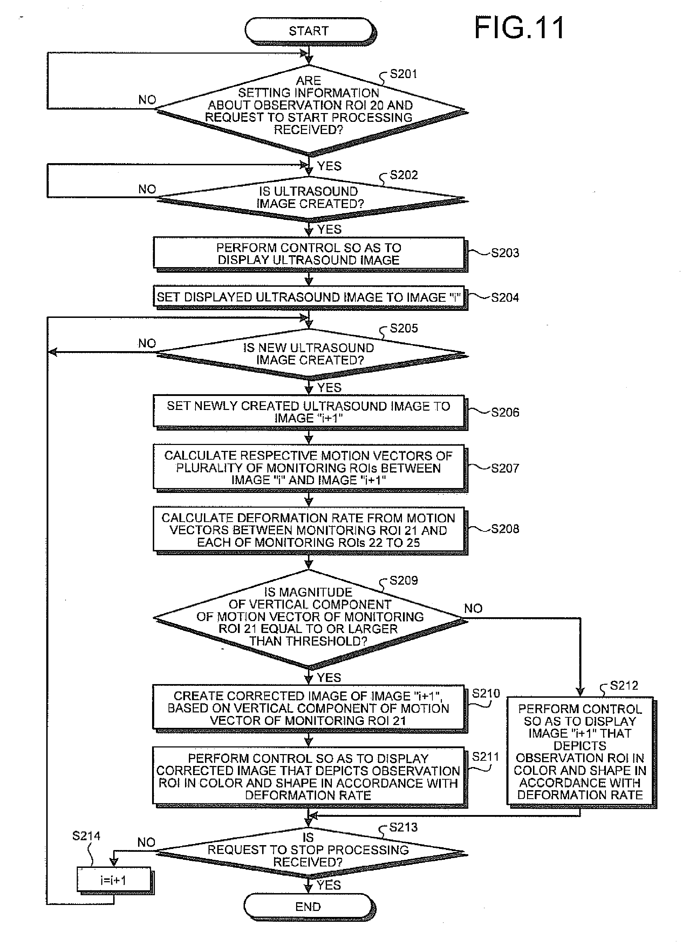

Processing by the ultrasound diagnosis apparatus according to the second embodiment is explained below with reference to FIG. 11. FIG. 11 is a flowchart for explaining the processing by the ultrasound diagnosis apparatus according to the second embodiment. With FIG. 11, processing to be performed after preliminarily setting a monitoring ROI and a threshold those are used for creation of a corrected image is explained below.

As shown in FIG. 11, the ultrasound diagnosis apparatus according to the second embodiment determines whether setting information about the observation ROI 20 and a request to start the processing are received (Step S201). In other words, the ultrasound diagnosis apparatus according to the second embodiment determines whether the operator draws the observation ROI 20 on a B-mode image with a drawing function of the input device 3, and the ROI positional-information acquiring unit 15d acquires positional information about the observation ROI 20 drawn on the B-mode image. Additionally, the ultrasound diagnosis apparatus according to the second embodiment determines whether the operator turns ON the "processing execution switch" included in the input device 3. If the setting information about the observation ROI 20 and the request to start the processing are not received (No at Step S201), the ultrasound diagnosis apparatus turns into a standby state.

By contrast, if the setting information about the observation ROI 20 and the request to start the processing are received (Yes at Step S201), the control unit 17 determines whether an ultrasound image is created by the image creating unit 15a (Step S202). The motion-vector calculating unit 15b sets a plurality of monitoring ROIs (the monitoring ROIs 21 to 25 shown in FIG. 9) in the observation ROI, based on the positional information about the observation ROI 20 acquired by the ROI positional-information acquiring unit 15d.

If ultrasound image is not created (No at Step S202), the control unit 17 is on standby until an ultrasound image is created. By contrast, if an ultrasound image is created (Yes at Step S202), the control unit 17 performs control so as to display the created ultrasound image on the monitor 2 (Step S203); and sets the displayed image to an image "i" (Step S204).

The control unit 17 then determines whether a new ultrasound image is created (Step S205); if new image is not created (No at Step S205), the control unit 17 is on standby until a new ultrasound image is created.

By contrast, if a new ultrasound image is created (Yes at Step S205), the control unit 17 sets the newly created ultrasound image to an image "i+1" (Step S206); and the motion-vector calculating unit 15b calculates respective motion vectors of a plurality of monitoring ROIs between the image "i" and the image "i+1" (Step S207).

The deformation-rate calculating unit 15e then calculates the deformation rate of the observation ROI 20 on the image "i+1" from the motion vectors of the monitoring ROI 21 and each of the monitoring ROIs 22 to 25 (Step S208). In other words, the deformation-rate calculating unit 15e calculates the length "a" of the long side and the length "b" of the short side of the observation ROI 20, and a compression rate of the observation ROI.

The corrected-image creating unit 15c then determines whether a magnitude of the vertical component of the motion vector of the monitoring ROI 21 calculated by the motion-vector calculating unit 15b is equal to or larger than the threshold (Step S209). If the magnitude of the vertical component of the motion vector of the monitoring ROI 21 is smaller than the threshold (No at Step S209), the control unit 17 performs control so as to display on the monitor 2 the image "i+1" that depicts the observation ROI in color and shape in accordance with the deformation rate (Step S212).

By contrast, if the magnitude of the vertical component of the motion vector of the monitoring ROI 21 is equal to or larger than the threshold (Yes at Step S209), the corrected-image creating unit 15c creates a corrected image of the image "i+1" based on the vertical component of the motion vector of the monitoring ROI 21 (Step S210). The control unit 17 then performs control so as to display on the monitor 2 the corrected image of the image "i+1" that depicts the observation ROI in color and shape in accordance with the deformation rate (Step S211).

After that, the control unit 17 determines whether the operator turns OFF the "processing execution switch" included in the input device 3, and a request to stop the processing is received (Step S213). If the request to stop the processing is not received (No at Step S213), the control unit 17 sets the image "i+1" to an image "i" (Step S214), returns to Step S205, and determines whether a new ultrasound image is created. In other words, the control unit 17 performs control such that the processing is to be executed between the ultrasound image set as an image "i" at Step S214, and an image "i+1" that is a new image set as an image "i+1" at Step S206.

By contrast, if the request to stop the processing is received (Yes at Step S213), the control unit 17 terminates the processing by the motion-vector calculating unit 15b, the corrected-image creating unit 15c, and the deformation-rate calculating unit 15e.

As described above, according to the second embodiment, the ROI positional-information acquiring unit 15d acquires positional information about the observation ROI 20 drawn by the operator on an ultrasound image; and the motion-vector calculating unit 15b sets a plurality of monitoring ROIs (the monitoring ROIs 21 to 25), based on the positional information about the observation ROI 20 on the ultrasound image acquired by the ROI positional-information acquiring unit 15d. The motion-vector calculating unit 15b calculates respective motion vectors of the monitoring ROIs; and the corrected-image creating unit 15c creates a corrected image by using the motion vector of the monitoring ROI 21 that is set at a substantial center of the observation ROI 20. The deformation-rate calculating unit 15e then calculates the deformation rate of the observation ROI 20 from the respective motion vectors of the monitoring ROIs 22 to 25 other than the monitoring ROI 21. For example, the deformation-rate calculating unit 15e calculates the deformation rate of the observation ROI 20 based on the motion vector of the monitoring ROI 21, and the respective motion vectors of the monitoring ROIs 22 to 25 other than the monitoring ROI 21.

Therefore, according to the second embodiment, visibility of an observation target on an ultrasound image can be improved; and an index indicating change in shape of the observation ROI 20 can be easily calculated from a B-mode image that is usually used, without using, such as the ultrasound elastography method.

Moreover, according to the second embodiment, the control unit 17 performs control so as to change the color tone of the observation ROI 20 in the image displayed on the monitor 2, based on the deformation rate (compression rate) of the observation ROI 20 calculated by the deformation-rate calculating unit 15e. Furthermore, according to the second embodiment, the control unit 17 performs control so as to deform the observation ROI 20 in the image displayed on the monitor 2, based on the deformation rate (the lengths of the long side and the short side) of the observation ROI 20 calculated by the deformation-rate calculating unit 15e. Therefore, according to the second embodiment, the operator can recognize change in shape of an observation target as the observation ROI drawn by the operator is deformed, and can further recognize the degree of change in shape of the observation target with the color tone of the observation ROI.

According to the second embodiment, the control unit 17 can control the monitor 2 so as to display the lengths of the long side and the short side calculated by the deformation-rate calculating unit 15e, or the compression rate. Also through such processing, the operator can recognize change in shape of an observation target.

The above description explains a case where the observation ROI 20 is set so as to include a border of a tumoral lesion of a breast. However, as a target for which the observation ROI 20 is set, there are various cases, for example, a tumoral lesion observed in a thyroid gland, a cyst observed in a breast, a blood vessel, a tendon, and the like.

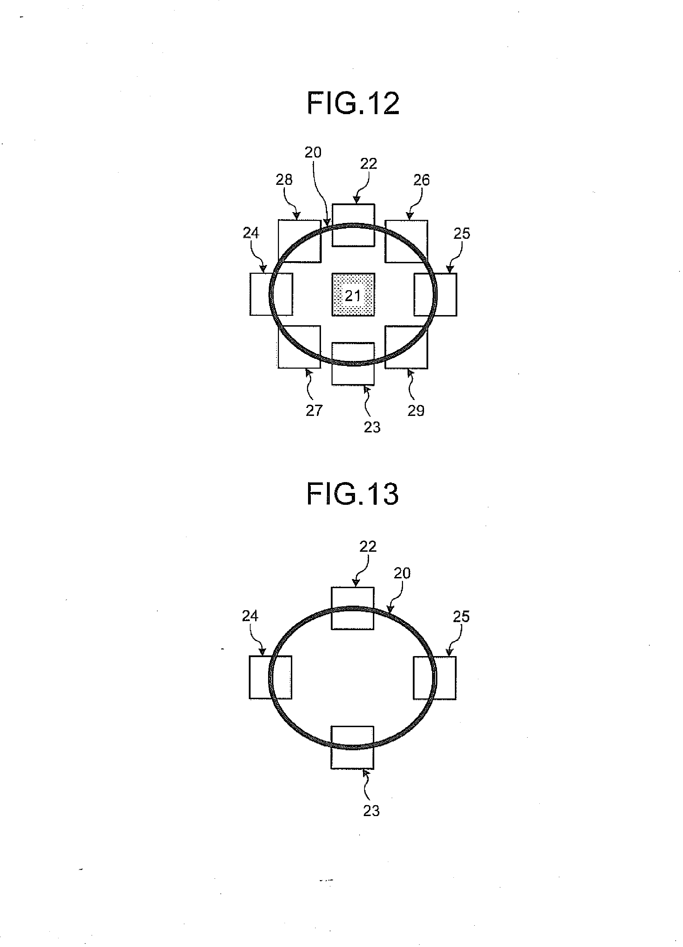

Moreover, the above description explains a case where the four monitoring ROIs 22 to 25 are set as local regions other than the first local region. However, the number of monitoring ROIs to be set as local regions other than the first local region can be arbitrarily set. FIG. 12 is a schematic diagram for explaining a first modification of the second embodiment.

For example, the motion-vector calculating unit 15b according to the first modification of the second embodiment sets the monitoring ROIs 22 to 25 at respective positions at top/bottom and right/left of the border of the observation ROI 20, as shown in FIG. 12. Furthermore, the motion-vector calculating unit 15b according to the first modification of the second embodiment sets monitoring ROIs 26 to 29, as shown in FIG. 12, in addition to the four monitoring ROIs 22 to 25. The monitoring ROIs 26 and 27 are set at respective positions at which, for example, a line running through the monitoring ROI 21 in the same direction as the direction of the motion vector of the monitoring ROI 21 crosses the border of the observation ROI 20. Moreover, the monitoring ROIs 28 and 29 are set at respective positions at which, for example, a line running through the monitoring ROI 21 in a direction perpendicular to the direction of the motion vector of the monitoring ROI 21 crosses the border of the observation ROI 20.

In other words, the motion-vector calculating unit 15b according to the first modification of the second embodiment sets the monitoring ROIs 26 to 29 in accordance with the direction of a force actually applied by the ultrasound probe 1 onto the living body tissue, in addition to setting the monitoring ROIs 22 to 25. Accordingly, the deformation-rate calculating unit 15e can calculate a more accurate deformation rate. Moreover, the motion-vector calculating unit 15b according to the first modification of the second embodiment can set only the monitoring ROIs 26 to 29.

Furthermore, the above description explains a case where a blur in the vertical direction is corrected by using the motion vector of the first local region, and the deformation rate of the observation ROI 20 is calculated by using the motion vectors of local regions other than the first local region. However, the second embodiment can be applied to a case where correction of blur in the vertical direction by using the motion vector of the first local region is not performed.

In other words, when a region of interest is drawn on an ultrasound image, the motion-vector calculating unit 15b according to a second modification of the second embodiment calculates respective motion vectors of a plurality of local regions set in the region of interest between a first image and a second image that are two successive ultrasound images in time series, among a plurality of ultrasound images created by the image creating unit 15a. The deformation-rate calculating unit 15e according to the second modification of the second embodiment then calculates the deformation rate of the region of interest based on the respective motion vectors of the local regions calculated by the motion-vector calculating unit 15b.

Specifically, according to the second modification of the second embodiment, a plurality of monitoring ROI is set on the border of the observation ROI 20. FIG. 13 is a schematic diagram for explaining the second modification of the second embodiment.

For example, according to the second modification of the second embodiment, as shown in FIG. 13, only the monitoring ROIs 22 to 25 are set at four positions that are the two end points of the long side and the two end points on the short side in the observation ROI 20 in ellipse. The motion-vector calculating unit 15b then calculates respective motion vectors of the monitoring ROIs 22 to 25; and the deformation-rate calculating unit 15e calculates a deformation rate of the observation ROI 20 based on the respective motion vectors of the monitoring ROIs 22 to 25.

Also according to the second modification of the second embodiment, by calculating the deformation rate of the observation ROI 20, for example, as shown in FIG. 10C, an image on which the shape and the color of a monitoring ROI vary can be displayed. According to the second modification of the second embodiment, because the blur in the vertical direction is not corrected, the observation ROI displayed on the ultrasound image is sometimes moved in the vertical direction in some cases; however, information about the deformation rate of the observation ROI can be provided to the operator. Therefore, also according to the second embodiment, visibility of an observation target on an ultrasound image can be improved.

The first and the second embodiments described above explain a case where calculation of motion vector is performed based on similarity in an image of an observation ROI. However, calculation of motion vector can be performed, for example, by using velocity information about tissue doppler.

In other words, the doppler processing unit 14 that can execute the tissue doppler method can analyze change in phase of reflected wave data with respect to each transmission pulse, thereby being capable to calculate velocity information at multiple points in living body tissue inside the subject P. Accordingly, the motion-vector calculating unit 15b can calculate a movement distance (motion vector) of a monitoring ROI between frames by multiplying the velocity value calculated by the doppler processing unit 14 with respect to each frame by a time between the frames. By using such method, a processing time by the motion-vector calculating unit 15b can be reduced.

Moreover, the first and the second embodiments can be applied to a case where a doppler image by the tissue doppler method is subjected to the image processing by the image processing unit 15. In other words, although a tumoral lesion is difficult to be identified because its boundary and the presence itself are unclear on a B-mode image; if its deformation rate is different from that of peripheral tissue, it is imaged in different color on an image by the tissue doppler method, so that the presence of the tumoral lesion can be easily confirmed. Accordingly, by subjecting a doppler image by the tissue doppler method to the image processing by the image processing unit 15, an image diagnosis by a doctor can be further assisted.

Moreover, the first and the second embodiments described above explain a case of performing the image processing by the image processing unit 15 when performing a diagnosis of a tumoral lesion. However, the image processing explained in the first and the second embodiments can be performed in a case of a general examination using ultrasound images. In other words, even when living body tissue of an observation target is moved because the body surface of the subject is pressed along with movement of the ultrasound probe 1, the ultrasound diagnosis apparatus can display ultrasound images on which the position of the observation target in the vertical direction is substantially constant, as a result, visibility of the observation target on the ultrasound images can be improved.

Furthermore, the first and the second embodiments described above explain a case of performing image processing in real time onto ultrasound images sequentially created along a time sequence. However, the processing by the image processing unit 15 is not limited to a case of being executed in real time along with creation of ultrasound image, and can be in a case of being executed by reading a plurality of ultrasound images in time series stored by the image memory 16.

Moreover, the first and the second embodiments described above explain a case of performing image processing onto an ultrasound image. However, the processing according to the first and the second embodiments can be applied to a case where it is performed by an image processing apparatus separately provided from the ultrasound diagnosis apparatus. Specifically, the image processing according to the first and the second embodiments can be executed by an image processing apparatus that includes functions of the image processing unit 15 other than the function of the image creating unit 15a shown in FIG. 2 or 7, by receiving a plurality of ultrasound images in time series from an ultrasound diagnosis apparatus, or a database of Picture Archiving and Communication System (PACS), which is a system of managing data of various medical images, or a database of electronic patients' medical record system that manages electronic patients' medical records attached with medical images.

The image processing method explained according to the first and the second embodiments can be implemented by executing a preliminarily prepared image processing program by an image processing apparatus that is a computer, such as a personal computer or a work station. The image processing program can be distributed via a network, such as the Internet. Moreover, the image processing program can be recorded onto a computer-readable recording medium, such as a hard disk, a Flexible Disk (FD), a Compact Disk Read Only Memory (CD-ROM), a magneto-optical disk (MO), and a Digital Versatile Disk (DVD); and can be executed by being read from a recording medium by an image processing apparatus that is a computer.

The components of each device shown in the drawings are conceptual for describing functions, and not necessarily to be physically configured as shown in the drawings. In other words, concrete forms of distribution and integration of the units are not limited to those shown in the drawings, and all or part of the units can be configured to be functionally or physically distributed and integrated in an arbitrary unit depending on various loads and conditions in use. Furthermore, all or an arbitrary part of processing functions performed by the respective units can be implemented by a Central Processing Unit (CPU) and a computer program to be executed by the CPU, or can be implemented as hardware by wired logic.

As explained above, according to the first and the second embodiments, visibility of an observation target on an ultrasound image can be improved.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

Further aspects:
1. An image processing apparatus comprising:
   a calculating unit (15b) adapted to calculate respective motion vectors of a plurality of local regions set in a region of interest between a first image and a second image that are two successive ultrasound images in time series among a plurality of ultrasound images that is created along a time sequence based on a reflected wave of ultrasound that is transmitted onto a subject from an ultrasound probe (1), when the region of interest is drawn on an ultrasound image; and
   a deformation-rate calculating unit (15e) adapted to calculate a deformation rate of the region of interest based on the respective motion vectors of the local regions calculated by the calculating unit (15b).
2. An ultrasound diagnosis apparatus comprising:
   an image creating unit (15a) adapted to create a plurality of ultrasound images in time series based on a reflected wave of ultrasound that is transmitted onto a subject from an ultrasound probe (1); and
   an image processing apparatus according to aspect 1.
3. The ultrasound diagnosis apparatus according to aspect 2, further comprising a display control unit (17) adapted to perform control so as to display on a certain display unit (2) a deformation rate of the region of interest calculated by the deformation-rate calculating unit (15e).
4. The ultrasound diagnosis apparatus according to aspect 2 or 3, wherein the display control unit (17) is adapted to perform control so as to change a color tone of a region of interest in an image displayed on the certain display unit (2), based on a deformation rate of the region of interest calculated by the deformation-rate calculating unit (15e).
5. The ultrasound diagnosis apparatus according to any of aspects 2 to 4, wherein the display control unit (17) is adapted to perform control so as to deform a region of interest in an image displayed on the certain display unit (2), based on a deformation rate of the region of interest calculated by the deformation-rate calculating unit (15e).
6. The ultrasound diagnosis apparatus according to any of aspects 2 to 5, further comprising a corrected-image creating unit (15c) adapted to create a corrected image corrected from the second image based on a component of a scanning line direction of the ultrasound in a motion vector of a first local region present at a certain position in the region of interest among the respective motion vectors of the local regions calculated by the calculating unit (15b), wherein the display control unit (17) is adapted to perform control so as to cause a certain display unit (2) to display the corrected image created by the corrected-image creating unit (15c).
7. The ultrasound diagnosis apparatus according to aspect 6, wherein
   when a magnitude of a component of a scanning line direction of the ultrasound in the motion vector is equal to or larger than a threshold, the corrected-image creating unit (15c) is adapted to create a corrected image from the second image, and
   when a corrected image is not created from the second image by the corrected-image creating unit (15c), the display control unit (17) is adapted to perform control so as to cause the certain display unit (2) to display the second image.
8. The ultrasound diagnosis apparatus according to aspect 6 or 7, wherein when receiving a request to execute processing from an operator via a certain input unit (3), the calculating unit (15b) and the corrected-image creating unit (15c) are adapted to execute calculation processing of the motion vector and creation processing of the corrected image, and when receiving a request to stop processing from the operator via the certain input unit (3), the calculating unit (15b) and the corrected-image creating unit (15c) are adapted to stop the calculation processing of the motion vector and the creation processing of the corrected image.
9. The ultrasound diagnosis apparatus according to any of aspects 2 to 8, further comprising a velocity-information calculating unit (14) adapted to calculate velocity information about living body tissue inside the subject based on the reflected wave, wherein the calculating unit (15b) is adapted to calculate the motion vector based on the velocity information calculated by the velocity-information calculating unit (14).
10. An image processing method comprising:
   calculating by a calculating unit (15b) respective motion vectors of a plurality of local regions set in a region of interest between a first image and a second image that are two successive ultrasound images in time series, among a plurality of ultrasound images that is created along a time sequence based on a reflected wave of ultrasound that is transmitted onto a subject from an ultrasound probe (1), when the region of interest is drawn on an ultrasound image; and
   calculating by a deformation-rate calculating unit (15e) a deformation rate of the region of interest based on the respective motion vectors of the local regions calculated by the calculating unit (15b).
11. An image processing apparatus comprising:
   a calculating unit (15b) adapted to calculate respective motion vectors of a plurality of local regions set in a region of interest between a first image and a second image that are two successive ultrasound images in time series among a plurality of ultrasound images that is created along a time sequence based on a reflected wave of ultrasound that is transmitted onto a subject from an ultrasound probe (1), when the region of interest is drawn on an ultrasound image;
   a corrected-image creating unit (15c) adapted to create a corrected image corrected from the second image based on a component of a scanning line direction of the ultrasound in a motion vector of a first local region present at a certain position in the region of interest among the respective motion vectors of the local regions calculated by the calculating unit (15b);
   a deformation-rate calculating unit (15e) adapted to calculate a deformation rate of the region of interest based on respective motion vectors of local regions other than the first local region; and
   a display control, unit (17) adapted to perform control so as to cause a certain display unit (2) to display the corrected image created by the corrected-image creating unit (15c).
12. An ultrasound diagnosis apparatus comprising:
   an image creating unit (15a) adapted to create a plurality of ultrasound images in time series based on a reflected wave of ultrasound that is transmitted onto a subject from an ultrasound probe (1); and
   an image processing apparatus according to aspect 11.
13. The ultrasound diagnosis apparatus according to aspect 12, wherein the display control unit (17) is adapted to perform control so as to display on the certain display unit (2) a deformation rate of the region of interest calculated by the deformation-rate calculating unit (15e).
14. The ultrasound diagnosis apparatus according to aspect 12 or 13, wherein the display control unit (17) is adapted to perform control so as to change a color tone of a region of interest in an image displayed on the certain display unit (2), based on a deformation rate of the region of interest calculated by the deformation-rate calculating unit (15e).
15. The ultrasound diagnosis apparatus according to any of aspects 12 to 14, wherein the display control unit (17) is adapted to perform control so as to deform a region of interest in an image displayed on the certain display unit (2), based on a deformation rate of the region of interest calculated by the deformation-rate calculating unit (15e).
16. The ultrasound diagnosis apparatus according to any of aspects 12 to 15, wherein
   when a magnitude of a component of a scanning line direction of the ultrasound in the motion vector is equal to or larger than a threshold, the corrected-image creating unit (15c) is adapted to create a corrected image from the second image, and
   when a corrected image is not created from the second image by the corrected-image creating unit (15c), the display control unit (17) is adapted to perform control so as to cause the certain display unit (2) to display the second image.
17. The ultrasound diagnosis apparatus according to any of aspects 12 to 16, wherein when receiving a request to execute processing from an operator via a certain input unit (3), the calculating unit (15b) and the corrected-image creating unit (15c) are adapted to execute calculation processing of the motion vector and creation processing of the corrected image, and when receiving a request to stop processing from the operator via the certain input unit (3), the calculating unit (15b) and the corrected-image creating unit (15c) are adapted to stop the calculation processing of the motion vector and the creation processing of the corrected image.
18. The ultrasound diagnosis apparatus according to any of aspects 12 to 17, further comprising a velocity-information calculating unit (14) adapted to calculate velocity information about living body tissue inside the subject based on the reflected wave, wherein the calculating unit (15b) is adapted to calculate the motion vector based on the velocity information calculated by the velocity-information calculating unit (14).
19. An image processing method comprising:
   calculating by a calculating unit (15b) respective motion vectors of a plurality of local regions set in a region of interest between a first image and a second image that are two successive ultrasound images in time series, among a plurality of ultrasound images that is created along a time sequence based on a reflected wave of ultrasound that is transmitted onto a subject from an ultrasound probe (1), when the region of interest is drawn on an ultrasound image;
   creating by a corrected-image creating unit (15c) a corrected image corrected from the second image based on a component of a scanning line direction of the ultrasound in a motion vector of a first local region present at a certain position in the region of interest among the respective motion vectors of the local regions calculated by the calculating unit (15b);
   calculating by a deformation-rate calculating unit (15e) a deformation rate of the region of interest based on respective motion vectors of local regions other than the first local region; and
   performing control by a display control unit (17) so as to cause a certain display unit (2) to display the corrected image created by the corrected-image creating unit (15c).

## Claims

1. An image processing apparatus comprising:
a calculating unit (15b) adapted to calculate a motion vector of a local region between a first image and a second image that are two successive ultrasound images in time series among a plurality of ultrasound images that is created along a time sequence based on a reflected wave of ultrasound that is transmitted onto a subject from an ultrasound probe (1);
a corrected-image creating unit (15c) adapted to create a corrected image corrected from the second image, based on a component of a scanning line direction of the ultrasound in the motion vector calculated by the calculating unit (15b); and
a display control unit (17) adapted to perform control so as to cause a certain display unit (2) to display the corrected image created by the corrected-image creating unit (15c).

2. An ultrasound diagnosis apparatus comprising:
an image creating unit (15a) adapted to create a plurality of ultrasound images in time series based on a reflected wave of ultrasound that is transmitted onto a subject from an ultrasound probe (1); and
an image processing apparatus according to claim 1.

3. The ultrasound diagnosis apparatus according to claim 2, wherein
when a magnitude of a component of a scanning line direction of the ultrasound in the motion vector is equal to or larger than a threshold, the corrected-image creating unit (15c) is adapted to create a corrected image from the second image, and
when a corrected image is not created from the second image by the corrected-image creating unit (15c), the display control unit (17) is adapted to perform control so as to cause the certain display unit (2) to display the second image.

4. The ultrasound diagnosis apparatus according to claim 2 or 3, wherein when receiving a request to execute processing from an operator via a certain input unit (3), the calculating unit (15b) and the corrected-image creating unit (15c) are adapted to execute calculation processing of the motion vector and creation processing of the corrected image, and when receiving a request to stop processing from the operator via the certain input unit (3), the calculating unit (15b) and the corrected-image creating unit (15c) stop the calculation processing of the motion vector and the creation processing of the corrected image.

5. The ultrasound diagnosis apparatus according to any of claims 2 to 4, wherein when a region of interest is drawn on an ultrasound image,
the calculating unit (15b) is adapted to calculate respective motion vectors of a plurality of local regions set in the region of interest,
the corrected-image creating unit (15c) is adapted to create the corrected image by using a motion vector of a first local region present at a certain position in the region of interest among the respective motion vectors of the local regions calculated by the calculating unit (15b), and
the ultrasound diagnosis apparatus further includes a deformation-rate calculating unit (15e) adapted to calculate a deformation rate of the region of interest based on respective motion vectors of local regions other than the first local region.

6. The ultrasound diagnosis apparatus according to claim 5, wherein the display control unit (17) is adapted to perform control so as to display on the certain display unit (2) a deformation rate of the region of interest calculated by the deformation-rate calculating unit (15e),

7. The ultrasound diagnosis apparatus according to claim 5 or 6, wherein the display control unit (17) is adapted to perform control so as to change a color tone of a region of interest in an image displayed on the certain display unit (2), based on a deformation rate of the region of interest calculated by the deformation-rate calculating unit (15e).

8. The ultrasound diagnosis apparatus according to any of claims 5 to 7, wherein the display control unit (17) is adapted to perform control so as to deform a region of interest in an image displayed on the certain display unit (2), based on a deformation rate of the region of interest calculated by the deformation-rate calculating unit (15e).

9. The ultrasound diagnosis apparatus according to any of claims 2 to 8, further comprising a velocity-information calculating unit (14) adapted to calculate velocity information about living body tissue inside the subject based on the reflected wave, wherein the calculating unit (15b) is adapted to calculate the motion vector based on the velocity information calculated by the velocity-information calculating unit (14).

10. An image processing method comprising:
calculating by a calculating unit (15b) a motion vector of a local region between a first image and a second image that are two successive ultrasound images in time series among a plurality of ultrasound images that is created along a time sequence based on a reflected wave of ultrasound that is transmitted onto a subject from an ultrasound probe (1);
creating by a corrected-image creating unit (15c) a corrected image corrected from the second image, based on a component of a scanning line direction of the ultrasound in the motion vector calculated by the calculating unit (15b); and
performing control by a display control unit (17) so as to cause a certain display unit (2) to display the corrected image created by the corrected-image creating unit (15c).
